# EUROPÄISCHE PATENTANMELDUNG

(11) **EP 3 795 405 A1**
(43) Veröffentlichungstag der Anmeldung: **24.03.2021**
(21) Anmeldenummer: 20197067.0
(22) Anmeldetag: 18.09.2020
(51) Int. Cl.: B60K 28/06, B60W 40/08, A61B 5/08

(54) **TRANSPORTVORRICHTUNG MIT EINER INSASSENKABINE UMFASSEND MINDESTENS EINEN SENSOR ZUR ATEMGASANALYSE, MINDESTENS EINE POSITIONSBESCHIMMUNGSVORRICHTUNG UND MINDESTENS EINE DATENVERARBEITUNGSVORRICHTUNG**

(30) Priorität: 20.09.2019 DE 102019125443
(71) Anmelder: VitaScale GmbH, 90449 Nürnberg (DE)
(72) Erfinder: Jerichow, Ulrich, 63571 Gelnhausen (DE)
(74) Vertreter: Metten, Karl-Heinz

(57) **Zusammenfassung**

Die Erfindung betrifft eine Transportvorrichtung mit einer Insassenkabine, umfassend eine Steuereinrichtung, eine Transportvorrichtungstür, ein Transportvorrichtungsdach, einen Sicherheitsgurt, einen Transportvorrichtungssitz, ein Headset, eine Kopfstütze, einen Rückspiegel, eine Sonnenblende und/oder ein Armaturenbrett, sowie ferner umfassend a) einen Sensor zur Atemgasanalyse, b) eine Positionsbestimmungsvorrichtung zur Ermittlung des Abstands zwischen dem Sensor und der Kopfvorderseite eines Kabineninsassens, c) eine Datenverarbeitungsvorrichtung und d) gegebenenfalls einen Sensor zur Messung mindestens eines Vitalparameters, wobei der eine Sensor zur Atemgasanalyse und gegebenenfalls der Sensor zur Messung eines Vitalparameters an oder in der Steuereinrichtung, an oder in der Transportvorrichtungstür, an oder in dem Transportvorrichtungsdach, an oder in dem Sicherheitsgurt, an oder in dem Transportvorrichtungssitz, an oder in dem Headset, an oder in der Kopfstütze, an oder in dem Rückspiegel, an oder in der Sonnenblende und/oder an oder in dem Armaturenbrett vorliegt, und wobei die Datenverarbeitungsvorrichtung mit einer Vorrichtung zum Blockieren der Transportvorrichtung und/oder einer Informationsausgabeeinheit in Wirkverbindung steht.

## Beschreibung

Die vorliegende Erfindung betrifft eine Transportvorrichtung mit einer Insassenkabine umfassend mindestens einen Sensor zur Atemgasanalyse, mindestens eine Positionsbestimmungsvorrichtung zur Ermittlung des Abstands zwischen dem Sensor und der Kopfvorderseite eines Kabineninsassens und mindestens eine Datenverarbeitungsvorrichtung.

Das Bedienen und Führen von Transportvorrichtungen erfordert höchste Konzentration. Gerade im Falle von weiteren Verkehrsteilnehmern, wie beispielsweise im Straßen- oder Schifffahrtsverkehr ist es unabdingbar, dass die Person an der Steuervorrichtung einer Transportvorrichtung ein ungetrübtes Urteilsvermögen besitzt, um angemessen auf eventuelle Gefahrensituationen oder eine wechselnde Verkehrslage reagieren zu können. Eine immer noch nicht außer Acht zu lassende Ursache von Verkehrsunfällen ist der Einfluss von Alkohol und anderen Drogen und das dadurch getrübte Urteilsvermögen der Verkehrsteilnehmer. Aber auch Schläfrigkeit, die beispielsweise aus der Überschreitung von Lenkzeiten herrührt, kann zu Verkehrsunfällen führen. Alkoholtester und Fahrzeugführer-Überwachungssysteme zur Vermeidung von Unfällen sind aus dem Stand der Technik hinreichend bekannt.

Eine Reihe von Systemen zeigt die Überwachung von Vitalparametern der Fahrzeugführer auf. Die DE 100 18 669 B4 offenbart eine Fahrer-Zustandsüberwachungseinrichtung zum Überprüfen des physischen Zustands eines Fahrers mit einem in einem Steuerrad angeordnet Pulssensor aus elektrisch leitendem Material. Dieser Pulssensor erzeugt durch Kontakt mit der Haut des Fahrers Pulssignale, welche mit Referenzdaten im Normalzustand des Fahrers abgeglichen und über eine Anzeigevorrichtung oder Audiosignale dem Fahrer mitgeteilt werden. Ein ähnliches System beschreibt die DE 10 2004 036 119 B4 mit einem Fahrerassistenzsystem zur Müdigkeitserkennung und Aufmerksamkeitsbeurteilung des Fahrzeugführers. Durch kontinuierliche Erfassung der Pulsfrequenz soll hierbei drohender einsetzender Sekundenschlaf erkannt werden, wobei bei plötzlicher Änderung der Pulsfrequenz eine Warnvorrichtung aktiviert wird, die akustische und optische Warnsignale abgibt. Weiterhin soll eine integrierte Speichervorrichtung bei einem eventuellen Eintreten eines Unfalls Aussagen über die mögliche Schuld des Fahrers zulassen können. Ein Nachteil solcher Systeme ist neben der Ungenauigkeit und der Fehlerbehaftung auch, dass keine Aussage über einen eventuellen Alkoholkonsum des Fahrzeugführers getroffen werden kann.

Alternative Systeme setzen auf die Bestimmung des Alkoholpegels eines Fahrzeugführers gleich zu Beginn der Fahrt, wie das System in der DE 10 2014 004 961 A1. Die dort offenbarte Vorrichtung zeigt ein Handgerät mit Mundstück zur Aufnahme einer Atem- oder Speichelprobe sowie einer Analyseeinheit zur Bestimmung der Alkoholkonzentration. Die Vorrichtung ist dabei mit einer Wegfahrsperre des Fahrzeugs gekoppelt, die den Start bzw. die Inbetriebnahme des Fahrzeugs verhindert. Nachteil eines solchen Handgeräts ist, dass die Atemgasüberwachung nur einmal zu Fahrtantritt durchgeführt werden kann, eine kontinuierliche Überwachung ist mit einem Handgerät eher wenig praktikabel aufgrund einer fehlenden Hand an der Steuervorrichtung und dem von der Fahrbahn abweichenden Blick auf das Display des Handgeräts.

Weitere Systeme ermitteln die Konzentration der Luft im Fahrzeuginnenraum. Die DE 10 2018 200 003 A1 zeigt beispielsweise ein Verfahren zur Atemanalyse von Fahrzeuginsassen, wobei ein zeitlicher Verlauf einer Konzentration eines Atemgases im Fahrzeuginnenraum ermittelt und ausgewertet wird. Weiterhin sollen mit dem Verfahren auch Maßnahmen zur Beeinflussung der Fahrzeuginsassen ergriffen werden können. Nachteile solcher Systeme sind meist die über den gesamten Fahrzeuginnenraum gemittelten Messungen. Es wird keine Unterscheidung zwischen Atemluft und Raumluft gemacht.

Bestehende Systeme zur Kontrolle und Überwachung der Fahrtüchtigkeit eines Fahrers lassen noch stets Wünsche offen. Der vorliegenden Erfindung lag daher die Aufgabe zugrunde, den Zustand von Fahrzeuginsassen, insbesondere hinsichtlich Fahrtüchtigkeit und Fahrtauglichkeit zuverlässig und während der gesamten Nutzungsdauer des Fahrzeugs ermitteln und kontrollieren zu können, und zwar vorzugsweise ohne dabei den Fahrzeugführer und/oder die Fahrzeuginsassen zu beeinträchtigen.

Demgemäß wurde eine Transportvorrichtung mit einer Insassenkabine gefunden, umfassend eine Steuereinrichtung, insbesondere Lenkrad oder Lenksäule, mindestens eine Transportvorrichtungstür, ein Transportvorrichtungsdach, mindestens einen Sicherheitsgurt, mindestens einen Transportvorrichtungssitz, ein, insbesondere kabelloses, Headset, mindestens eine Kopfstütze, mindestens einen Rückspiegel, mindestens eine verfahrbare, insbesondere klappbare Sonnenblende, und/oder ein Armaturenbrett, insbesondere eine Steuereinrichtung, vorzugsweise Lenkrad oder Lenksäule, mindestens eine Transportvorrichtungstür, ein Transportvorrichtungsdach und mindestens einen Transportvorrichtungssitz sowie gegebenenfalls mindestens einen Sicherheitsgurt, mindestens einen Rückspiegel, mindestens eine verfahrbare, insbesondere klappbare Sonnenblende, mindestens eine Kopfstütze, mindestens ein, insbesondere kabelloses, Headset und/oder ein Armaturenbrett, ferner umfassend
a) mindestens einen Sensor, insbesondere eine Vielzahl an Sensoren, zur Atemgasanalyse,
b) mindestens eine Positionsbestimmungsvorrichtung zur Ermittlung des Abstands zwischen dem Sensor und der Kopfvorderseite eines Kabineninsassens, insbesondere des Kabineninsassens an der Steuereinrichtung, und
c) mindestens eine Datenverarbeitungsvorrichtung,
wobei der mindestens eine Sensor zur Atemgasanalyse an oder in der Steuereinrichtung, an oder in der Transportvorrichtungstür, an oder in dem Transportvorrichtungsdach, an oder in dem Sicherheitsgurt, an oder in dem Transportvorrichtungssitz, an oder in dem Headset, an oder in der Kopfstütze, an oder in dem Rückspiegel, an oder in der Sonnenblende und/oder an oder in dem Armaturenbrett vorliegt, und wobei die Datenverarbeitungsvorrichtung mit einer Vorrichtung zum Blockieren der Transportvorrichtung und/oder einer Informationsausgabeeinheit in Wirkverbindung steht oder bringbar ist. In einer bevorzugten Ausführungsform verfügt die erfindungsgemäße Transportvorrichtung mit zusätzlich zu dem mindestens einen Sensor zur Atemgasanalyse ferner über d) mindestens einen Sensor zur Messung mindestens eines Vitalparameters. Dieser Sensor zur Messung mindestens eines Vitalparameters kann dabei ebenfalls an oder in der Steuereinrichtung, an oder in der Transportvorrichtungstür, an oder in dem Transportvorrichtungsdach, an oder in dem Sicherheitsgurt, an oder in dem Transportvorrichtungssitz, an oder in dem Headset, an oder in der Kopfstütze, an oder in dem Rückspiegel, an oder in der Sonnenblende und/oder an oder in dem Armaturenbrett vorliegen. In einer Ausführungsform ist es möglich, dass der Sensor für die Atemgasanalyse und der Sensor für die Messung mindestens eines Vitalparameters an dem selben Ort vorliegen, beispielsweise an oder in der Steuereinrichtung. Auch kann vorgesehen sein, dass der Sensor für die Atemgasanalyse und der Sensor für die Messung mindestens eines Vitalparameters in einer kombinierten Sensoreinheit vorliegen. In einer weiteren Ausgestaltung kann vorgesehen sein, dass der Sensor für die Atemgasanalyse ebenfalls dafür ausgelegt und eingerichtet ist, gegebenenfalls in Kombination mit der Datenverarbeitungsvorrichtung, mindestens einen Vitalparametern zu bestimmen.

In einer zweckmäßigen Ausgestaltung der erfindungsgemäßen Transportvorrichtung ist vorgesehen, dass der mindestens eine Sensor, insbesondere die Vielzahl an Sensoren, zur Atemgasanalyse ausgelegt und eingerichtet ist, um mindestens einen der Stoffe ausgewählt aus der Gruppe bestehend aus Alkoholen, insbesondere Ethanol, Ketonen, insbesondere Aceton, Aldehyden, Carbonsäuren, Kohlenstoffoxiden, Stickstoffoxiden, Sauerstoff, Wasserstoff, Wasser und Mischungen dieser Stoffe qualitativ und quantitativ zu identifizieren. Über die Bestimmung des Ethanol-Gehaltes kann festgestellt werden, ob dem Fahrzeugführer noch gestaltet werden kann, die Transportvorrichtung in Betrieb zu nehmen. Wird zum Beispiel auf die Bestimmung von Wasserstoff und/oder Ketonen abgestellt, sind Rückschlüsse auf Stoffwechselprobleme möglich, welche gegebenenfalls auch der Inbetriebnahme und/oder der Führung der Transportvorrichtung entgegenstehen. Dies kann in Wirkverbindung mit der Datenverarbeitungsvorrichtung je nach vorgegebener Einstellung von Grenzwerten bzw. Grenzbereichen zur Aktivierung der Vorrichtung zum Blockieren der Transportvorrichtung und/oder einer Informationsausgabeeinheit führen.

Demgemäß kann vorgesehen sein, dass der mindestens eine Sensor, insbesondere die Vielzahl an Sensoren zur Atemgasanalyse, ein Alkoholsensor ist oder diesen umfasst und/oder dass der mindestens eine Sensor, insbesondere die Vielzahl an Sensoren zur Atemgasanalyse, ausgelegt und eingerichtet ist, um Motorverbrennungsgase zu detektieren.

Auch hat sich als besonders zweckmäßig erwiesen, wenn der mindestens eine Sensor, insbesondere die Vielzahl an Sensoren zur Atemgasanalyse, ausgelegt und eingerichtet ist, um den Atemrhythmus, die Atemzugtiefe und die Atemhäufigkeit der Kabineninsassen der Transportvorrichtung, insbesondere des Kabineninsassens an der Steuereinrichtung, zu ermitteln. Dieses gestattete zum Beispiel die zuverlässige Verstellung des Ermüdungsgrades eines Fahrzeugführers.

Ferner sind derartige erfindungsgemäße Transportvorrichtungen bevorzugt, bei denen der mindestens eine Sensor, insbesondere die Vielzahl an Sensoren zur Atemgasanalyse, ausgelegt und eingerichtet ist, um über eine Vielzahl an Atemzügen, insbesondere über nur einen Atemzug, besonders bevorzugt einen Ruheatemzug, eines Kabineninsassens der Transportvorrichtung, insbesondere des Kabineninsassens an der Steuereinrichtung, eine Atemgasanalyse durchzuführen. Mit der erfindungsgemäßen Transportvorrichtung gelingt es, mithilfe nur eines Atemzuges die Analyse des Atemgases durchzuführen, um darüber Rückschlüsse auf zum Beispiel die Fahrtauglichkeit eines Fahrers zu erlangen.

In einer weiteren bevorzugten Ausführungsform der erfindungsgemäßen Transportvorrichtung kann vorgesehen sein, dass der mindestens eine Sensor, insbesondere die Vielzahl an Sensoren zur Atemgasanalyse, Bestandteil eines Headsets oder Sensormoduls ist. Hierbei ist besonders bevorzugte eine solche Transportvorrichtung, bei der das Headset oder Sensormodul aus der Steuereinrichtung, der Transportvorrichtungstür, dem Transportvorrichtungsdach, dem Sicherheitsgurt, dem Transportvorrichtungssitz, der Kopfstütze, dem Rückspiegel, der Sonnenblende und/oder dem Armaturenbrett, insbesondere reversibel, herausfahrbar ist, wobei dieses besonders bevorzugt aus der Steuereinrichtung, der Transportvorrichtungstür, dem Transportvorrichtungsdach, der Kopfstütze, dem Rückspiegel, der Sonnenblende und/oder dem Armaturenbrett reversibel herausfahrbar ist.

Der mindestens eine Sensor zur Messung mindestens eines Vitalparameters kann zweckmäßigerweise ausgelegt und eingerichtet sein, um den Puls, die Herzfrequenz, die Hautleitfähigkeit, die Sauerstoffsättigung des Blutes, die Schweißmenge und/oder die Schweißzusammensetzung eines Kabineninsassens, insbesondere des Kabineninsassens an der Steuereinrichtung, zu ermitteln.

Mit der erfindungsgemäßen Transportvorrichtung gelingt es in einer Ausführungsform ferner, mit dem mindestens einen Sensor zur Atemgasanalyse und/oder dem mindestens einen Sensor zur Messung mindestens eines Vitalparameters kabineninsassenspezifische Messungen durchzuführen.

Dabei kann zweckmäßigerweise vorgesehen sein, dass die mindestens eine Positionsbestimmungsvorrichtung zur Ermittlung des Abstands zwischen Sensor und der Kopfvorderseite eines Kabineninsassens, insbesondere des Kabineninsassens an der Steuereinrichtung, mindestens eine Kamera und/oder ein Infrarotsystem umfasst.

In einer weiteren Ausgestaltung kann die erfindungsgemäße Transportvorrichtung ferner mindestens einen Temperatursensor ausgelegt und eingerichtet, um die Temperatur in der Insassenkabine zu ermitteln, und/oder mindestens einen Luftfeuchtigkeitssensor ausgelegt und eingerichtet, um die Luftfeuchtigkeit in der Insassenkabine zu ermitteln, und/oder mindestens einen Sensor ausgelegt und eingerichtet, um die die Feuchtigkeit in der ausgeatmeten Atemluft eines Kabineninsassens, insbesondere des Kabineninsassens an der Steuereinrichtung, zu ermitteln, und/oder mindestens einen Strömungssensor ausgelegt und eingerichtet, um die Luftströmung in der Insassenkabine zu ermitteln, insbesondere bei eingeschalteter Belüftung oder Heizvorrichtung, umfassen.

In einer besonders zweckmäßigen Ausführungsform der erfindungsgemäßen Transportvorrichtung ist vorgesehen, dass die Sensoren, insbesondere der Sensor zur Atemgasanalyse und/oder der Sensoren zur Messung des mindestens einen Vitalparameters, ausgelegt und eingerichtet sind, um Messwerte, insbesondere die Alkoholkonzentration, zu Beginn eines Transports und/oder in bestimmten zeitlichen Intervallen während eines Transports eines Kabineninsassens, insbesondere des Kabineninsassens an der Steuereinrichtung, zu ermitteln und diese an die Datenverarbeitungsvorrichtung zu übermitteln. Hierbei hat sich als vorzugsweise erwiesen, wenn die Datenverarbeitungsvorrichtung ausgelegt und eingerichtet ist, um die Messwerte mit hinterlegten Referenzwerten abzugleichen. Dabei ist die Datenverarbeitungsvorrichtung vorzugsweise derart ausgelegt und eingerichtet, um bei Ermittlung eines über einem hinterlegten Grenzwert liegenden Wertes die Vorrichtung zum Blockieren der Transportvorrichtung und/oder die Informationsausgabeeinheit zu aktivieren.

Die Informationsausgabeeinheit kann in einer Ausgestaltung in der Transportvorrichtung vorliegen. Alternativ oder zusätzlich kann die Informationsausgabeeinheit auch außerhalb der Transportvorrichtung vorliegen und beispielsweise kabellos mit der Datenverarbeitungsvorrichtung verbunden oder verbindbar sein, beispielsweise mit einem Krankenhaus, einer Notarztstation, einer Polizeistation und dergleichen.

Des Weiteren haben sich solche erfindungsgemäßen Transportvorrichtungen als besonders geeignet erwiesen, bei denen zusätzlich mindestens eine Kameraeinheit, insbesondere in Wirkverbindung mit der Datenverarbeitungsvorrichtung, vorliegt, die ausgelegt und eingerichtet ist zur Gesichtserkennung des Fahrers der Transportvorrichtung und/oder mindestens eines Insassen in der Transportvorrichtung und/oder zur Erkennung der Position von Mund und/oder Nase relativ zu dem mindestens einen Sensor.

Die erfindungsgemäße Transportvorrichtung kann zum Beispiel ausgewählt sein aus der Gruppe bestehend aus Personenkraftfahrzeugen, Lastkraftfahrzeugen, Bussen, Bahnen, Schiffen, Flugzeugen, Gabelstaplern, Motorrädern, insbesondere dreirädrigen Motorrädern, und landwirtschaftliche Maschinen.

Mit der erfindungsgemäßen Transportvorrichtung gelingt es überraschenderweise besonders zuverlässig, den Zustand der Fahrtauglichkeit eines Insassen einer Transportvorrichtung, insbesondere des Fahrzeugführers, zu bestimmen und in Reaktion auf die ermittelten und analysierten Daten mithilfe einer Datenverarbeitungsvorrichtung den oder die Insassen, insbesondere den Fahrzeugführer auf mögliche Gefahren und Beeinträchtigungen beim Führen der Transportvorrichtung direkt oder indirekt hinzuweisen bzw. die Inbetriebnahme oder das Fortsetzen der Betätigung der Transportvorrichtung zu unterbinden. Auf diese Weise können sehr zielgerichtet mögliche Gefahrensituationen, zum Beispiel ein zu hoher Alkoholgehalt im Blut oder eine eintretende Ermüdung, frühzeitig erkannt und die Gefahr von Verletzungen von Mensch und Umwelt signifikant reduziert werden.

Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Transportvorrichtung mit einer Insassenkabine, umfassend eine Steuereinrichtung, insbesondere Lenkrad oder Lenksäule, mindestens eine Transportvorrichtungstür, ein Transportvorrichtungsdach, mindestens einen Sicherheitsgurt, mindestens einen Transportvorrichtungssitz, ein, insbesondere kabelloses, Headset, mindestens eine Kopfstütze, mindestens einen Rückspiegel, mindestens eine verfahrbare, insbesondere klappbare Sonnenblende, und/oder ein Armaturenbrett, insbesondere eine Steuereinrichtung, vorzugsweise Lenkrad oder Lenksäule, mindestens eine Transportvorrichtungstür, ein Transportvorrichtungsdach und mindestens einen Transportvorrichtungssitz sowie gegebenenfalls mindestens einen Sicherheitsgurt, mindestens einen Rückspiegel, mindestens eine verfahrbare, insbesondere klappbare Sonnenblende, mindestens eine Kopfstütze, mindestens ein, insbesondere kabelloses, Headset und/oder ein Armaturenbrett, ferner umfassend
a) mindestens einen Sensor, insbesondere eine Vielzahl an Sensoren, zur Atemgasanalyse,
b) mindestens eine Positionsbestimmungsvorrichtung zur Ermittlung des Abstands zwischen dem Sensor und der Kopfvorderseite eines Kabineninsassens, insbesondere des Kabineninsassens an der Steuereinrichtung,
c) mindestens eine Datenverarbeitungsvorrichtung und
d) gegebenenfalls mindestens einen Sensor zur Messung mindestens eines Vitalparameters,
wobei der mindestens eine Sensor zur Atemgasanalyse und gegebenenfalls der Sensor zur Messung mindestens eines Vitalparameters
an oder in der Steuereinrichtung, an oder in der Transportvorrichtungstür, an oder in dem Transportvorrichtungsdach, an oder in dem Sicherheitsgurt, an oder in dem Transportvorrichtungssitz, an oder in dem Headset, an oder in der Kopfstütze, an oder in dem Rückspiegel, an oder in der Sonnenblende und/oder an oder in dem Armaturenbrett vorliegt, und wobei die Datenverarbeitungsvorrichtung mit einer Vorrichtung zum Blockieren der Transportvorrichtung und/oder einer Informationsausgabeeinheit in Wirkverbindung steht oder bringbar ist.

2. Transportvorrichtung nach Anspruch 1, **dadurch gekennzeichnet, dass**
der mindestens eine Sensor, insbesondere die Vielzahl an Sensoren, zur Atemgasanalyse ausgelegt und eingerichtet ist, um mindestens einen der Stoffe ausgewählt aus der Gruppe bestehend aus Alkoholen, insbesondere Ethanol, Ketonen, insbesondere Aceton, Aldehyden, Carbonsäuren, Kohlenstoffoxiden, Stickstoffoxiden, Sauerstoff, Wasserstoff, Wasser und Mischungen dieser Stoffe qualitativ und quantitativ zu identifizieren.

3. Transportvorrichtung nach Anspruch 2, **dadurch gekennzeichnet, dass**
der mindestens eine Sensor, insbesondere die Vielzahl an Sensoren zur Atemgasanalyse, ein Alkoholsensor ist oder diesen umfasst und/oder dass der mindestens eine Sensor, insbesondere die Vielzahl an Sensoren zur Atemgasanalyse, ausgelegt und eingerichtet ist, um Motorverbrennungsgase zu detektieren.

4. Transportvorrichtung nach einem der vorangehenden Ansprüche, **dadurch gekennzeichnet, dass**
der mindestens eine Sensor, insbesondere die Vielzahl an Sensoren zur Atemgasanalyse, ausgelegt und eingerichtet ist, um den Atemrhythmus, die Atemzugtiefe und die Atemhäufigkeit der Kabineninsassen der Transportvorrichtung, insbesondere des Kabineninsassens an der Steuereinrichtung, zu ermitteln.

5. Transportvorrichtung nach einem der vorangehenden Ansprüche, **dadurch gekennzeichnet, dass**
der mindestens eine Sensor, insbesondere die Vielzahl an Sensoren zur Atemgasanalyse, ausgelegt und eingerichtet ist, um über eine Vielzahl an Atemzügen, insbesondere über nur einen Atemzug, besonders bevorzugt einen Ruheatemzug, eines Kabineninsassens der Transportvorrichtung, insbesondere des Kabineninsassens an der Steuereinrichtung, eine Atemgasanalyse durchzuführen.

6. Transportvorrichtung nach einem der vorangehenden Ansprüche, **dadurch gekennzeichnet, dass**
der mindestens eine Sensor, insbesondere die Vielzahl an Sensoren zur Atemgasanalyse, Bestandteil eines Headsets oder Sensormoduls ist, insbesondere eines aus der Steuereinrichtung, der Transportvorrichtungstür, dem Transportvorrichtungsdach, dem Sicherheitsgurt, dem Transportvorrichtungssitz, der Kopfstütze, dem Rückspiegel, der Sonnenblende und/oder dem Armaturenbrett reversibel herausfahrbaren Headsets oder Sensormoduls, besonders bevorzugt eines aus der Steuereinrichtung, der Transportvorrichtungstür, dem Transportvorrichtungsdach, der Kopfstütze, dem Rückspiegel, der Sonnenblende und/oder dem Armaturenbrett reversibel herausfahrbaren Headsets oder Sensormoduls, ist.

7. Transportvorrichtung nach einem der vorangehenden Ansprüche, **dadurch gekennzeichnet, dass**
der mindestens eine Sensor zur Messung mindestens eines Vitalparameters ausgelegt und eingerichtet ist, um den Puls, die Herzfrequenz, die Hautleitfähigkeit, die Sauerstoffsättigung des Blutes, die Schweißmenge und/oder die Schweißzusammensetzung eines Kabineninsassens, insbesondere des Kabineninsassens an der Steuereinrichtung, zu ermitteln.

8. Transportvorrichtung nach einem der vorangehenden Ansprüche, **dadurch gekennzeichnet, dass**
der mindestens eine Sensor zur Atemgasanalyse und/oder der mindestens eine Sensor zur Messung mindestens eines Vitalparameters ausgelegt und eingerichtet ist, um kabineninsassenspezifische Messungen durchzuführen.

9. Transportvorrichtung nach einem der N-orangehenden Ansprüche, **dadurch gekennzeichnet, dass**
die mindestens eine Positionsbestimmungsvorrichtung zur Ermittlung des Abstands zwischen Sensor und der Kopfvorderseite eines Kabineninsassens, insbesondere des Kabineninsassens an der Steuereinrichtung, mindestens eine Kamera und/oder ein Infrarotsystem umfasst.

10. Transportvorrichtung nach einem der vorangehenden Ansprüche, ferner umfassend mindestens einen Temperatursensor ausgelegt und eingerichtet, um die Temperatur in der Insassenkabine zu ermitteln und/oder
mindestens einen Luftfeuchtigkeitssensor ausgelegt und eingerichtet, um die Luftfeuchtigkeit in der Insassenkabine zu ermitteln und/oder die Feuchtigkeit in der ausgeatmeten Atemluft eines Kabineninsassens, insbesondere des Kabineninsassens an der Steuereinrichtung zu ermitteln, und/oder
mindestens einen Strömungssensor ausgelegt und eingerichtet, um die Luftströmung in der Insassenkabine zu ermitteln, insbesondere bei eingeschalteter Belüftung oder Heizvorrichtung.

11. Transportvorrichtung nach einem der vorangehenden Ansprüche, **dadurch gekennzeichnet, dass**
die Sensoren, insbesondere der Sensor zur Atemgasanalyse und/oder der Sensoren zur Messung des mindestens einen Vitalparameters, ausgelegt und eingerichtet sind, um Messwerte, insbesondere die Alkoholkonzentration, zu Beginn eines Transports und/oder in bestimmten zeitlichen Intervallen während eines Transports eines Kabineninsassens, insbesondere des Kabineninsassens an der Steuereinrichtung, zu ermitteln und diese an die Datenverarbeitungsvorrichtung zu übermitteln,
wobei vorzugsweise die Datenverarbeitungsvorrichtung ausgelegt und eingerichtet ist, um die Messwerte mit hinterlegten Referenzwerten abzugleichen.

12. Transportvorrichtung nach einem der vorangehenden Ansprüche, **dadurch gekennzeichnet, dass**
die Datenverarbeitungsvorrichtung ausgelegt und eingerichtet ist, um bei Ermittlung eines über einem hinterlegten Grenzwert liegenden Wertes die Vorrichtung zum Blockieren der Transportvorrichtung und/oder die Informationsausgabeeinheit zu aktivieren.

13. Transportvorrichtung nach Anspruch 12, **dadurch gekennzeichnet, dass**
die Informationsausgabeeinheit in der Transportvorrichtung und/oder außerhalb der Transportvorrichtung vorliegt.

14. Transportvorrichtung nach einem der vorangehenden Ansprüche, **gekennzeichnet durch**
mindestens eine Kameraeinheit, insbesondere in Wirkverbindung mit der Datenverarbeitungsvorrichtung, ausgelegt und eingerichtet zur Gesichtserkennung des Fahrers der Transportvorrichtung und/oder mindestens eines Insassen in der Transportvorrichtung und/oder zur Erkennung der Position von Mund und/oder Nase relativ zu dem mindestens einen Sensor.

15. Transportvorrichtung nach einem der vorangehenden Ansprüche, **dadurch gekennzeichnet, dass**
die Transportvorrichtung ausgewählt ist aus einer Gruppe bestehend aus Personenkraftfahrzeugen, Lastkraftfahrzeugen, Bussen, Bahnen, Schiffen, Gabelstapler, Flugzeugen, Motorrädern, insbesondere dreirädrigen Motorrädern, und landwirtschaftliche Maschinen.
